# EUROPEAN PATENT SPECIFICATION

(11) **EP 1 137 414 B1**
(45) Date of publication and mention of the grant of the patent: **12.10.2005**
(21) Application number: 99967223.1
(22) Date of filing: 08.12.1999
(51) Int. Cl.: A61K 31/44, A61K 31/445, A61K 31/195

(54) **USE OF ACETYLCHOLINESTERASE INHIBITORS ON THE MODULATION OF THE HYPOTHALAMIC-PITUITARY-GONADAL AXIS**
VERWENDUNG VON ACETYLCHOLINESTERASE INHIBITOREN ZUR MODULIERUNG DER HYPOTHALAMUS-HYPOPHYSEN-GONADENACHSE
UTILISATION D'INHIBITEURS D'ACETYLCHOLINE ESTERASE POUR LA MODULATION DE L'AXE HYPOTHALAMO-HYPOPHYSO-GONADIQUE

(30) Priority: 11.12.1998 US 111913 P
(43) Date of publication of application: 04.10.2001
(73) Proprietor: Davis, Bonnie M., Syosset, NY 11791 (US)
(72) Inventor: Davis, Bonnie M., Syosset, NY 11791 (US)
(74) Representative: Ebner von Eschenbach, Jennifer
(86) International application number: PCT/US1999/028972
(87) International publication number: WO 2000/033840

(56) References cited:
- US-A- 5 602 176
- HINZ, G. ET AL: "Long-term changes in sexual functions in rats treated neonatally with psychotropic drugs" DEV. ENDOCRINOL. (1978), 3(HORM. BRAIN DEV.), 121-7, XP001061916
- LAMBERTS, S. W. J. ET AL.: "Pituitary insufficiency" LANCET, vol. 352, 11 July 1998 (1998-07-11), pages 127-134, XP002196314

## Description

### Field of the Invention

The present invention relates to the use of centrally-acting cholinesterase inhibitors to modulate the hypothalamic-pituitary-gonadal axis by stimulating the secretion of gonadotropin releasing hormone. Such modulation finds use in, for example, stimulation of ovulation, in treatment of luteal phase defect, aesthenozoospermia, oligozoospermia, hypogonadotrophic hypogonadism and cryptorchidism and in induction of puberty.

### Background of the Invention

The purpose of this invention is to influence the secretion of gonadotropin releasing hormone (GnRH - may be known as GRF, previously referred to as LHRH or LRF) using orally-active preparations influencing central cholinergic activity.

The addition of acetylcholine to anterior pituitary glands incubated with hypothalamic fragments results in luteinizing hormone (LH) release, which can be blocked by muscarinic blockade by atropine. Prostigmine, an acetylcholinesterase inhibitor, as well as acetylcholine, enhance LH release in this system. (Fiorindo RP and Martini L, Evidence for a cholinergic component in the neuroendocrine control of luteinizing hormone secretion. Neuroendocrinology 18:322-332, 1975). Follicle stimulating hormone (FSH) levels are increased by the addition of hypothalamic fragments to incubated anterior pituitary glands, with a further increase produced by acetylcholine, an effect antagonized by atropine. (Simonovic I, Motta M and Martini L, Acetylcholine and the release of follicle stimulating hormone releasing factor. Endocrinology 95:1371, 1974) As cholinergic agents have no direct effect on anterior pituitaries to stimulate LH or FSH release, they were presumed to release gonadotropin-releasing hormone (GnRH, formerly thought to be two hormones, luteinizing hormone releasing hormone, LHRH, and follicle stimulating hormone releasing hormone, FSHRH) from the hypothalamus. Since the advent of assays for GnRH, this fact has been directly demonstrated. (Richardson SB, Prasad JA, Hollander CS, Acetylcholine, melatonin, and potassium depolarization stimulate release of luteinizing hormone-releasing hormone from rat hypothalamus in vitro. Proc Natl Acad Aci U S A 79(8):2686-9, 1982) In intact rats, injection of acetylcholine into the lateral ventricle stimulates LH release. This can be blocked with atropine or enhanced with prostigmine. Thus, cholinergic stimulation appears to cause GnRH release from the hypothalamus, apparently by a muscarinic mechanism.

Both muscarinic cholinergic blockade with atropine and nicotinic cholinergic stimulation appear to suppress gonadotropin release via hypothalamic mechanisms. Hemicastration in the female rat normally results in gonadotropin-mediated hypertrophy of the remaining ovary. This can be blocked by atropine implants into the anterior hypothalamus. (Monti JM, Sala MA, Otegui JE et al, Inhibition of ovarian compensatory hypertrophy by implants of atropine in the hypothalamus. Experientia 26:1263-1264, 1970) Similar implants blocked the post-orchidectomy rise in LH and FSH in male rats. No effect was seen of atropine implants into the pituitary, a finding consistent with the inability of acetylcholine to stimulate gonadotropin release from pituitary glands in vitro, as noted above. (Libertun C and McCann SM, Blockade of the postorchidectomy increase in gonadotropin by implants of atropine into the hypothalamus, Proc Soc Exp Biol Med 152:143-146, 1976) These data are consistent with the existence of a muscarinic cholinergic mechanism mediating GnRH release in the hypothalamus.

Conversely, nicotine administration suppresses LH secretion with consequent delay or inhibition of ovulation. (Blake CA, Parallelism and divergence in luteinizing and follicle stimulating hormone release in nicotine-treated rats. Proc Soc Exp Biol Med 145:716-720, 1974; Kanematsu S and Sawyer CH, Inhibition of the progesterone-advanced LH surge at proestrus. Proc Soc Exp Biol Med 143:1183-5, 1973; Blake CA, Scaramuzzi RJ, Norman RL, Kanematsu S and Sawyer CH, Nicotine delays the ovulatory surge of luteinizing hormone in the rat. Proc Soc Exp Biol Med 141:1014-1016, 1972) This action occurs within the hypothalamus, as complete surgical isolation does not alter it (Blake CA, Norman RL and Sawyer CH, Localization of the inhibitory actions of estrogen and nicotine on release of luteinizing hormone in rats. Neuroendocrinology 16:22-35, 1974) and it is not exerted at the pituitary, which is still fully responsive to exogenous LHRH. (Blake CA, Norman RL and Sawyer CH, Effects of hypothalamic deafferentation and ovarian steroids on pituitary responsiveness to LH-RH in female rats. In Gual and Rosemberg, eds, Hypothalamic hypophysiotropic hormones. Excerpta Medica, Amsterdam, 1973, pp. 33-38) Although nicotinic effects alone are inhibitory, the data indicate that the net effect of acetylcholinesterase inhibition or acetylcholine administration is to stimulate gonadotropin secretion in the short term.

### Summary of the Invention

The present invention provides a method of treatment of conditions that can benefit from stimulation of the hypothalamic-pituitary-gonadal axis such as failure of ovulation, some cases of unexplained infertility, luteal phase defect, hypogonadotrophic hypogonadism, aesthenozoospermia, oligozoospermia, delayed onset of puberty and cryptorchidism.

### Detailed Description of the Invention

Suitable acetylcholinesterase inhibitors for the present invention include those that have a central effect and have a medium to long duration of action (such as from 1 to 100 hours, typically from 1.5 to 72 hours). Suitable acetylcholinesterase inhibitors are those that will pass easily through the blood-brain barrier. Suitable compounds for this purpose include donepezil, rivastigmine, galantamine, lycoramine and the analogs of galantamine and lycoramine. The most suitable compounds for this purpose are galanthamine, lycoramine and their analogs wherein wherein at least one of the methoxy, hydroxy or methyl groups of the galanthamine or lycoramine is replaced as follows:
the methoxy group by another alkoxy group of from one to six carbon atoms, a hydroxy group, hydrogen, an alkanoyloxy group, a benzoyloxy or substituted benzoyloxy group, a carbonate group or a carbamate groupor a trialkylsilyloxy group;
the hydroxy group by an alkoxy group of from one to six carbon atoms, hydrogen, an alkanoyloxy group, a benzoyloxy or substituted benzoyloxy group, a carbonate group or a carbamate group;
the N-methyl group by hydrogen, alkyl, benzyl, cyclopropylmethyl group or a substituted or unsubstituted benzoyloxy group.

One or more of the methoxy, hydroxy and methyl groups of galanthamine or lycoramine may be replaced by the groups noted above.

When reference is made to a substituent group, said group may be selected from alkyl or alkoxy groups of from 1 to 6 carbon atoms, halo groups, and haloalkyl groups such as trifluoromethyl. When reference is made to alkyl groups, where the context permits, the term also includes groups which are or contain cycloalkyl groups including adamantyl. Aryl groups are typically phenyl or naphthyl but may include heteroatyl such as morpholino.

Galanthamine and lycoramine have the following formulae:

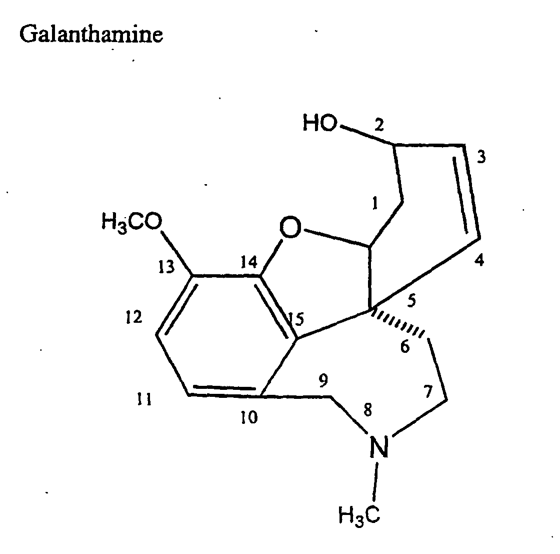

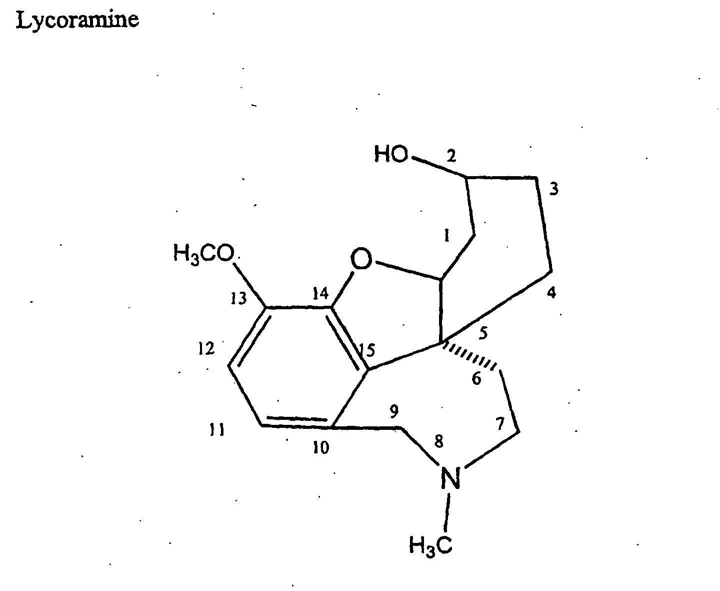

Suitable analogs are described for example in International Patent Publication WO88/08708 and an article by Bores and Kosley in Drugs of the Future 21: 621-631 (1996). Other useful pharmacologic agents for such preparations include rivastigmine, and other pharmacologic agents with half lives of 1-11 hours.

Particularly useful.analogs of galanthamine and lycoramine that are of use in the present invention include analogs thereof wherein the methoxy group of such compounds is replaced by a hydrogen, hydroxy or alkoxy group of from two to six carbon atoms or an acyloxy group, for example an alkanoyloxy of from one to seven carbon atoms or benzoyl group, or where the methoxy group is replaced by a mono or dialkyl carbamate or a mono or dialkyl carbonate group wherein the alkyl groups contain from 1 to 8 carbon atoms, preferably of from 4 to 8 carbon atoms or wherein the methoxy group thereof is replaced by an aryl carbamate or carbonate group wherein said aryl group is selected from phenyl, naphthyl, substituted phenyl and substituted naphthyl groups wherein said substituent is selected from alkyl and alkoxy groups of from 1 to 6 carbon atoms, trifluoro methyl groups and halo groups.

Other useful analogs include compounds wherein, independently of whether or not the methoxy group has been replaced, the hydroxy group is replaced by an alkoxy group of from one to six carbon atoms, hydrogen, an acyloxy group, for example an alkanoyloxy group, typically of from 1 to 7 carbon atoms, a benzoyloxy or substituted benzoyloxy group wherein said substituent is selected from alkyl and alkoxy groups of from 1 to 6 carbon atoms, trifluoro methyl groups and halo groups, a carbonate group or a carbamate group which may be a mono or dialkyl or an aryl carbamate or carbonate wherein the alkyl groups contain from 1 to 8 carbon atoms, preferably of from 4 to 6 carbon atoms or said aryl group is selected from phenyl, naphthyl, substituted phenyl and substituted naphthyl groups wherein said substituent is selected from alkyl and alkoxy groups of from 1 to 6 carbon atoms, trifluoromethyl groups and halo groups.

GnRH and various longer acting agonists are used in a wide variety of clinical situations. They may be used to stimulate or suppress the HPG axis. They may be used alone, or in combination with gonadotropic preparations, hormones, or hormone antagonists. Short-term (or pulsatile) administration of GnRH stimulates gonadotropin secretion, while continuous administration or the use of long acting analogs for more than approximately two weeks desensitizes the gonadotropes and decreases gonadotropin secretion. This "medical castration" has numerous applications. (Ascoli M and Segaloff DL, Adenohypophyseal hormones and their hypothalamic releasing factors, in Goodman and Gilman's The Pharmacological Basis of Therapeutics, 9th edition, McGraw-Hill, New York, 1996, pp 1363-1382)

The peptides used to influence the reproductive axis must be given by injection, infusion or intranasally. LH and FSH preparations, including HCG and HMG, are given by injection. The subject of the present invention is the use of moderate or long-acting, orally available, centrally active cholinergic agents to stimulate gonadotropin secretion, alone or in combination with gonadotropins, GnRH, GnRH analogs or other hormonal preparations. Oral cholinergic agents may, in suitable patients, substitute for parenteral medications such as testosterone preparations or gonadotropins. The use of these agents could avoid the need for injections, subcutaneous or intravenous infusion pumps, and the inconvenience and risk of infection. Manipulation of the HPG axis for ovulation induction at the level of the hypothalamus is more physiological than GnRH and gonadotropins. It may be expected to reduce the incidence of ovarian hyperstimulation syndrome and multiple gestations, because normal feedback inhibitory mechanisms can operate at the level of the pituitary gland and the hypothalamus, just as the use of GnRH, acting on the pituitary gland, decreased the incidence of these complications from that of gonadotropin therapy, which acts directly on the ovaries. (Blacker CM, Ovulation stimulation and induction. Endo Metab Clin N Am 21(1):57-83, 1992).

The preferred agents for use in the present invention are rivastigmine and galantamine. Donepezil may be used in certain situations. These are acetylcholinesterase inhibitors which are well absorbed orally and partition preferentially to brain.

Rivastigmine has a plasma half life of 1-2 hours and is administered in doses of 0.5 to 10 mg two to six times a day, with a maximum daily dose of 18 mg. (Product Monograph: Exelon, Novartis Pharma AG, Basel, Switzerland, April 1998) Galantamine has a plasma half-life of 5 hours and is administered in doses of 1-20 mg two to four times a day with a maximum dose of 60 mg a day. (Westra P, et al Pharmacokinetics of galantamine (a long-acting anticholinesterase drug) in anaesthetized patients. Br J Anaesth 58:1303-1307, 1986; Mihailova D, et al Pharmacokinetics of galantamine hydrobromide after single subcutaneous and oral dosage in humans. Pharmacology 39:50-58, 1989). Donepezil is given in doses of 1-15 mg once daily. (Rogers SL, et al A 24-week, double-blind, placebo-controlled trial of donepezil in patients with Alzheimer's disease. Neurology 50:136-145, 1998) Doses for children may be proportionately reduced, and controlled-release dosage forms may override multiple dose schedules.

Common side effects include nausea and vomiting, anorexia, dizziness, fatigue, aesthenia. They are lessened by slow buildup of dose, and decrease with continued administration. Donepezil produces a higher frequency of muscle cramps than the shorter acting drugs, and insomnia, which they do not produce.

Cholinergic agents may exacerbate depression. Cholinergic agents exert vagotonic effects on the heart, and thus may exacerbate the sick sinus syndrome or bradycardia and may cause bronchoconstriction, particularly in patients with asthma and chronic obstructive pulmonary disease, may increase gastrointestinal motility or acid secretion, may theoretically increase bladder motility, may cause seizures or worsen Parkinsonian symptoms, or the symptoms of organophosphate anticholinesterase poisoning. There are insufficient data on the teratogenicity to rule out possible fetal malformations, and data on the safe use of these compounds in children are needed as well.

### Owlation-Inducing Protocols

Many patients with hypogonadotropic amenorrhea or hypothalamic dysfunction who are properly evaluated and followed and are deemed candidates for infusion pump treatment with GnRH may respond to the oral administration of acetylcholinesterase inhibitors. Protocols vary considerably among centers with the use of stimulatory agents guided by rises in plasma hormone measurements and ultrasound determinations of ovarian follicle size. The evaluation and monitoring used for GnRH administration would be used with acetylcholinesterase inhibitor ovulation induction. In the protocol employed by Blacker, for example, (Blacker CM, Ovulation stimulation and induction, Endo Metab Clin N Am 21(1):57-83, 1992) therapy is begun at the beginning of the follicular phase with the intermediate-acting agents, galantamine at 2-5 mg tid or rivastigmine at 1-2 mg tid, or appropriate delayed-release dosage form of either compound. If a normal follicular response as determined by transvaginal ultrasound or serum estradiol levels is not obtained, the dose may be increased to a maximum of galantamine, 15-20 mg tid, or rivastigmine, 4-6 mg tid, as tolerated. If the response is excessive at any point, dosages must be reduced to prevent ovarian hyperstimulation or multiple gestation. In unusual cases, the doses may be increased to galantamine, 30-35 mg tid, or rivastigmine, 8 mg tid. The small decrease in plasma hormone levels immediately preceding ovulation may be reproduced by decreasing or withholding medication for a short period. Luteal support may be provided by continuing the galantamine or rivastigmine for approximately two weeks, preferably at lower doses. Alternatively, luteal support may be provided, as in Blacker's description, with GnRH infusion, HCG injections, or progesterone administration. Determination of a suitable dose may be accomplished by means known to those skilled in the art. It is, however, likely that the dose will vary between individuals and that it will only be established after several menstrual cycles.

Additional GnRH protocols have been described by Miller MM, Hoffman DI, Ovulation induction, in KL Becker, ed, Principles and Practice of Endocrinology and Metabolism JB Lippincott, Philadelphia, 1995, pp 900-909; Ascoli M and Segaloff DL, Adenohypophyseal hormones and their hypothalamic releasing factors, in Goodman and Gilman's The Pharmacological Basis of Therapeutics, 9th edition, McGraw-Hill, New York, 1996, pp 1363-1382; Fauser and Van Heusden, Endo Rev 18(1):71-106, 1997.

### Superovulation in the Treatment of Unexplained Infertility

Cholinergic agents stimulate gonadotropin secretion in short-term studies of experimental animals and are of use in promoting the development of additional follicles during normal menstrual cycles in infertility cases. Studies with clomiphene or HMG injection suggest an increase in observed pregnancy rates. (Deaton JL, Gibson M, Nakajima ST et al, A randomized controlled trial of clomiphene citrate and intrauterine insemination versus well-timed intercourse in couples with unexplained infertility or surgically corrected endometriosis, Fertil Steril 54:1083, 1990; Collins JA, Superovulation in the treatment of unexplained infertility. Semin Reprod Endocrinol 8:165, 1990) A protocol similar to ovulation induction may be followed, with treatment continuing into the luteal phase if required due to luteal phase insufficiency, or standard luteal phase therapies may be used.

### Luteal Phase Defect

Luteal phase dysfunction may be treated by acetylcholinesterase inhibitors of the present invention, using endometrial biopsies and serum progesterone concentrations to assess outcome. Treatment is begun at 2-5 mg galantamine bid or tid, or 1-2 mg rivastigmine tid, and increased every few days in small increments until the desired result is obtained. It will take several cycles to establish the correct dose. Correction of the luteal phase dysfunction may require initiating treatment during the follicular phase. (Rebar RW, Disorders of menstruation, ovulation and sexual response, in KL Becker, ed, Principles and Practice of Endocrinology and Metabolism, JB Lippincott, Philadelphia, 1995, pp 880-899)

### Hypogonadotropic Hypogonadism

Cholinesterase inhibitors of the present invention are of use for the stimulation of endogenous GnRH in patients who are potentially responsive such as women with functional hypothalamic amenorrhea, male acquired idiopathic hypogonadotropic hypogonadism, some cases of Kallman's syndrome or other hypothalamic dysfunction who may not have complete absence of GnRH cells. Initial doses of 2-5 mg of galantamine bid or tid or 1-2mg of rivastigmine could be raised by 4 mg of galantamine per day once a week, or 1-2 mg of rivastigmine once a day per week to a daily dose of 15-75 mg of galantamine or 8-18 mg of rivastigmine, with the final dose taken not later than late afternoon, to avoid continuous stimulation and subsequent downregulation. Alternatively, donepezil beginning at 5 mg/day, increasing to 10 mg/day after four weeks may be administered. Some patients may tolerate up to 15 mg/day. The lowest effective doses should be used.

The outcome measurements could, depending on the clinical objective and gender, include FSH or LH measurements, menstrual function, estradiol, progesterone or testosterone measurements, or sperm counts. Gonadotropin secretion should be monitored at intervals to ensure that stimulation has not been superseded by suppression after long-term treatment.

### Induction of Puberty

The treatment protocol is essentially the same as for hypogonadotropic hypogonadism. Patients whose gonads and pituitary glands are responsive to GnRH, after priming, may have hypothalamic function sufficient to respond to cholinergic therapy. Given that the expected treatment with GnRH would involve several years of use of a portable infusion pump, a trial of oral cholinergic therapy may be reasonable to assess its potential. Using dosing regimens described above for hypogonadotropic hypogonadism, and allowing for a two-month period to comfortably reach an optimal dose with minimal induction of side effects, followed by four months of therapy, a six-month trial would be recommended to assess the potential for efficacy in an individual patient. The optimal dose will be individualized as with GnRH. Outcome measures may include hormone or gonadotropin measurements, testicular volume, growth velocity and development of secondary sexual characteristics. Gonadotropin secretion should be monitored at intervals to ensure that stimulation has not been superseded by suppression after long-term treatment.

### Oligozoospermia and/or Aesthenozoospeimia

Infertile males with normal or abnormal gonadotropins and low sperm counts or abnormal sperm characteristics have been treated with GnRH preparations with improvements. (Aulitzky W, Frick J, Hadziselimovic F, Pulsatile LHRH therapy in patients with oligozoospermia and disturbed LH pulsatility. Int J Androl 12(4):265-72, 1989; Matsumiya K, Kitamura M, Kishikawa H, Kondoh N, Fujiwara Y, Okuyama A, A prospective comparative trial of a gonadotropin-releasing hormone analogue with clomiphene citrate for the treatment of oligoaesthenozoospermia. Int J Urol 5(4):361-3, 1998) The same treatment regimen as proposed for hypogonadotropic hypogonadism is used here. A minimum of three months therapy is necessary to evaluate effects on spenn.

### Cryptorchidism

Some cases of undescended testes respond to treatment with LHRH and/or HCG. (Lala R, Matarazzo P, Chiabotto P, Gennari F, Cortese MG, Canavese F, DeSanctis C, Early hormonal and surgical treatment of cryptorchidism. J Urol 157(5):1898-1901, 1997; Fedder J and Boesen M, Effect of a combined GnRH/hCG therapy in boys with undescended testicles: evaluated in relation to testicular localization within the first week after birth. Arch Androl 40(3)181-186, 1998) A trial of treatment of boys with cryptorchidism with cholinesterase inhibitors would follow the protocol proposed for hypogonadotrophic hypogonadism, with weight-based dosage adjustments.

Dosages set out above have been for galanthamine or rivastigmine. However, dosages for other suitable agents can be determined by standard techniques such as those set out for example in Chapter 6 (by Benjamin Calesnick) of Drill's Pharmacology in Medicine (Fourth Edition Joseph R DiPalma ed, McGraw-Hill 1971 or in Chapter 6 (by B. E. Rodda et al) of Biopharmaceutical Statistics for Drug Development (ed. Karl E. Peace, Marcel Dekker Inc, 1988).

## Claims

1. Use of an acetylcholinesterase inhibitor having a central effect and a duration of action of from 1 to 100 hours for preparation of a medicament for treatment of a condition that can benefit from stimulation of the hypothalamic-pituitary-gonadal axis wherein the condition is selected from failure of ovulation, unexplained fertility, luteal phase defect, hypogonadotrophic hypogonadism, aesthenozoospermia, oligozoospermia, delayed onset of puberty and cryptochidism and wherein said acetycholinesterase inhibitor is selected from donepezil, rivastigmine, galantamine, lycoramine and analogs of galantamine or lycoramine.

2. A use as claimed in claim 1, wherein said acetylcholinesterase inhibitor has a duration of action from 1.5 to 72 hours.

## Patentansprüche

1. Verwendung eines Acetylcholinesterasehemmers mit einer zentralen Wirkung und einer Wirkungsdauer von 1 bis 100 Stunden für die Herstellung eines Medikamentes zur Behandlung von eines Krankheitszustandes, die einen Nutzen ziehen kann aus der Stimulation der hypothalamischen-hypophysären-gonadialen Achse, wobei der Krankheitszustand ausgewählt ist aus: Ovulationsversagen, unerklärte Fertilität, Lutealphasedefekt, hypogonadotroper Hypogonadismus, Ästhenozoospermie, Oligozoospermie, Spätreife und Kryptorchismus; wobei der Acetylcholinesterasehemmer ausgewählt ist aus: Donepezil, Rivastigmin, Galantamin, Lycoramin sowie Analoga von Galantamin oder Lycoramin.

2. Verwendung nach Anspruch 1 wobei der Acetylcholinesterasehemmer eine Wirkungsdauer von 1,5 bis 72 Stunden hat.

## Revendications

1. Utilisation d'un inhibiteur d'acétylcholinestérase ayant un effet central et une durée d'action de 1 à 100 heures pour la préparation d'un médicament pour le traitement d'une affection qui peut bénéficier de la stimulation de l'axe hypothalamique-pituitaire-gonadique où l'affection est choisie parmi défaut d'ovulation, fécondité inexpliquée, défaut de phase lutéale, hypogonadisme hypogonadotrophique, asthénozoospermie, oligozoospermie, déclenchement retardé de la puberté et cryptochidisme et où ledit inhibiteur d'acétylcholinestérase est choisi parmi donepezil, rivastigmine, galantamine, lycoramine et les analogues de galantamine ou lycoramine.

2. Utilisation selon la revendication 1, où ledit inhibiteur d'acétylcholinestérase a une durée d'action de 1,5 à 72 heures.
